# EUROPEAN PATENT APPLICATION

(11) **EP 0 547 699 A1**
(43) Date of publication of application: **23.06.1993**
(21) Application number: 92203876.5
(22) Date of filing: 11.12.1992
(51) Int. Cl.: C07K 5/02, C07K 5/06, C07K 5/08, C07C 255/29, A61K 38/55

(54) **Peptidyl derivatives as inhibitors of interleukin-1B converting enzyme**

(30) Priority: 19.12.1991 US 811160
(71) Applicant: MERCK & CO. INC., Rahway New Jersey 07065-0900 (US)
(72) Inventor: Chapman, Kevin T., Scotch Plains, NJ 07076 (US); Maccoss, Malcolm, Freehold, NJ 07728 (US)
(74) Representative: Thompson, John Dr.

(57) **Abstract**

Novel peptidyl derivatives of formula I are found to be potent inhibitors of interleukin-1β converting enzyme (ICE). Compounds of formula I may be useful in the treatment of inflammatory or immune-based diseases of the lung and airways; central nervous system and surrounding membranes; the eyes and ears; joints, bones, and connective tissues; cardiovascular system including the pericardium; the gastrointestinal
and urogenital systems; the skin and mucosal membranes. Compounds of formula I are also useful in treating the complications of infection (e.g., gram negative shock) and tumors in which IL 1 functions as an autocrine growth factor or as a mediator of cachexia.

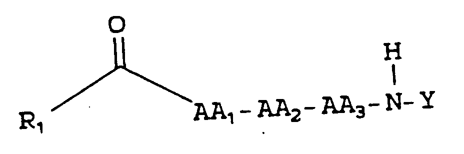

## Description

### BACKGROUND OF THE INVENTION

This invention relates to substituted peptidyl derivatives useful in the treatment of inflammation in lung, central nervous system, kidney, joints, endocardium, pericardium, eyes, ears, skin, gastrointestinal tract and urogenital system. More particularly, this invention relates substituted peptidyl lactones and open forms thereof that are useful inhibitors of interleukin-1β converting enzyme (ICE). Interleukin-1β converting enzyme (ICE) has been identified as the enzyme responsible for converting precursor interleukin-1β (IL-1β) to biologically active IL-1β.

Mammalian interleukin-1 (IL-1) is an immunoregulatory protein secreted by cell types as part of the inflammatory response. The primary cell type responsible for IL-1 production is the peripheral blood monocyte. Other cell types have also been described as releasing or containing IL-1 or IL-1 like molecules. These include epithelial cells (Luger, et al., J. Immunol. 127: 1493-1498 (1981), Le et al., J. Immunol. 138: 2520-2526 (1987) and Lovett and Larsen, J. Clin. Invest. 82: 115-122 (1988), connective tissue cells (Ollivierre et al., Biochem. Biophys. Res. Comm. 141: 904-911 (1986), Le et al, J. Immunol. 138: 2520-2526 (1987), cells of neuronal origin (Giulian et al., J. Esp. Med. 164: 594-604 (1986) and leukocytes (Pistoia et al., J. Immunol. 136: 1688-1692 (1986), Acres et al., Mol. Immuno. 24: 479-485 (1987), Acres et al., J. Immunol. 138: 2132-2136 (1987) and Lindenmann et al., J. Immunol 140: 837-839 (1988).

Biologically active IL-1 exists in two distinct forms, IL-1α with an isoelectric point of about pI 5.2 and IL-1β with an isoelectric point of about 7.0 with both forms having a molecular mass of about 17,500 (Bayne et al., J. Esp. Med. 163: 1267-1280 (1986) and Schmidt, J. Esp. Med. 160: 772 (1984). The polypeptides appear evolutionarily conserved, showing about 27-33% homology at the amino acid level (Clark et al., Nucleic Acids Res. 14: 7897-7914 (1986).

Mammalian IL-1β is synthesized as a cell associated precursor polypeptide with a molecular mass of about 31.4 kDa (Limjuco et al., Proc. Natl. Acad. Sci USA 83: 3972-3976 (1986). Precursor IL-1β is unable to bind to IL-1 receptors and is biologically inactive (Mosley et al., J. Biol. Chem. 262: 2941-2944 (1987). Biological activity appears dependent upon some form of proteolytic processing which results in the conversion of the precursor 31.5 kDa form to the mature 17.5 kDa form. Evidence is growing that by inhibiting the conversion of precursor IL-1β to mature IL-1β, one can effectively inhibit the activity of interleukin-1.

Mammalian cells capable of producing IL-1β include, but are not limited to, karatinocytes, endothelial cells, mesangial cells, thymic epithelial cells, dermal fibroblasts, chondrocytes, astrocytes, glioma cells, mononuclear phagocytes, granulocytes, T and B lymphocytes and NK cells.

As discussed by J.J. Oppenheim, et al. Immunology Today, vol. 7(2):45-56 (1986), the activities of interleukin-1 are many. It has been observed that catabolin, a factor that promotes degradation of cartilage matrix, also exhibited the thymocyte comitogenic activities of IL-1 and stimulates chondrocytes to release collagenase neutral proteases and plasminogen activator. In addition, a plasma factor termed proteolysis inducing factor stimulates muscle cells to produce prostaglandins which in turn leads to proteolysis, the release of amino acids and, in the long run, muscle wasting, and appears to represent a fragment of IL-1 with fever-inducing, acute phase response and thymocyte co-mitogenic activities.

IL-1 has multiple effects on cells involved in inflammation and wound healing. Subcutaneous injection of IL-1 leads to margination of neutrophils and maximal extravascular infiltration of the polymorphonuclear leukocytes (PMN). In vitro studies reveal IL-1 to be a chemotactic attractant for PMN to activate PMN to metabolize glucose more rapidly to reduce nitroblue tetrazolium and to release their lysozomal enzymes. Endothelial cells are stimulated to proliferate by IL-1 to produce thromboxane, to become more adhesive and to release procoagulant activity. IL-1 also enhances collagen type IV production by epidermal cells, induces osteoblast proliferation and alkaline phosphatase production and stimulates osteoclasts to resorb bone. Even macrophages have been reported to be chemotactically attracted to IL-1 to produce prostaglandins in response to IL-1 and to exhibit a more prolonged and active tumoricidal state.

IL-1 is also a potent bone resorptive agent capable upon infusion into mice of causing hypercaleemia and increas in bone resorptive surface as revealed by his to morphometry Sabatini, M. et al., PNAS 85: 5235-5239, 1988.

Accordingly, disease states in which the ICE inhibitors of Formula I may be useful as therapeutic agents include, but are not limited to, infectious diseases where active infection exists at any body site, such as meningitis and salpingitis;
complications of infections including septic shock, disseminated intravascular coagulation, and/or adult respiratory distress syndrome; acute or chronic inflammation due to antigen, antibody, and/or complement deposition; inflammatory conditions including arthritis, cholangitis, colitis, encephalitis, endocarditis, glomerulonephritis, hepatitis, myocarditis, pancreatitis, pericarditis, reperfusion injury and vasculitis. Immune-based diseases which may be responsive to ICE inhibitors of Formula I include but are not limited to conditions involving T-cells and/or macrophages such as acute and delayed hypersensitivity, graft rejection, and graft-versus-host-disease; auto-immune diseases including Type I diabetes mellitus and multiple sclerosis. ICE inhibitors of Formula I may also be useful in the treatment of bone and cartilage resorption as well as diseases resulting in excessive deposition of extracellular matrix. Such diseases include periodonate diseases interstitial pulmonary fibrosis, cirrhosis, systemic sclerosis, and keloid formation. ICE inhibitors of Formula I may also be useful in treatment of certain tumors which produce IL 1 as an autocrine growth factor and in preventing the cachexia associated with certain tumors.

### SUMMARY OF THE INVENTION

Novel peptidyl aldehydes, ring chain tautomers and hydrates thereof of formula I are found to be potent inhibitors of interleukin-1β converting enzyme (ICE). Compounds of formula I are useful in the treatment of deseases including inflammation in lung, central nervous system, kidney, joints, endocardium, pericardium, eyes, ears, skin, gastrointestinal tract and urogenital system.

### DETAILED DESCRIPTION OF THE INVENTION

The invention encompasses compounds of formula I. 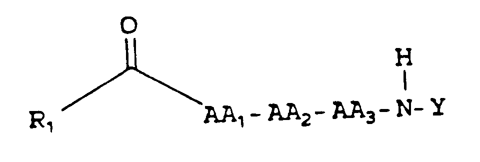
or a pharmaceutically acceptable salt thereof thereof: wherein Y is: 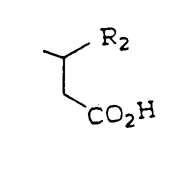
R₁ is
(a) substituted C₁₋₁₂ alkyl, wherein the substituent is selected from
   (1) hydrogen,
   (2) hydroxy,
   (3) halo, and
   (4) C₁₋₆alkylcarbonyl;
(b) aryl C₁₋₆ alkyl wherein the aryl group is selected from the group consisting of:
   (1) phenyl,
   (2) naphthyl,
   (3) pyridyl,
   (4) furyl,
   (5) thienyl,
   (6) thiazolyl,
   (7) isothiazolyl,
   (8) imidazolyl,
   (9) benzimidazolyl,
   (10) pyrazinyl,
   (11) pyrimidyl,
   (12) quinolyl,
   (13) isoquinolyl,
   (14) benzofuryl,
   (15) benzothienyl,
   (16) pyrazolyl,
   (17) indolyl,
   (18) purinyl,
   (19) isoxazolyl, and
   (20) oxazolyl,
and mono and di-substituted aryl as defined above in items (1) to (20) wherein the substitutents are independently C₁₋₆alkyl, halo, hydroxy, C₁₋₆alkyl amino, C₁₋₆alkoxy, C₁₋₆alkylthio, and C₁₋₆alkylcarbonyl;
R₂ is -C≡N
AA₁ is independently selected from the group consisting of
(a) a single bond, and
(b) an amino acid of formula AI 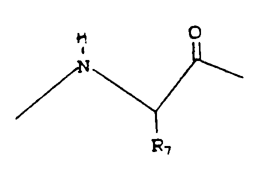
wherein R₇ is selected from the group consisting of:
(a) hydrogen,
(b) substituted C₁₋₆ alkyl, wherein the substituent is selected from
   (1) hydrogen,
   (2) hydroxy,
   (3) halo,
   (4) -S-C₁₋₄ alkyl
   (5) -SH
   (6) C₁₋₆ alkylcarbonyl,
   (7) carboxy,
   (8) 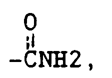
   (9) amino carbonyl amino,
   (10) C₁₋₄ alkylamino, wherein the alkyl moiety is substituted with hydrogen or hydroxy, and the amino is substituted with hydrogen or CBZ,
   (11) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl;
AA₂ is independently selected from the group consisting of
(a) a single bond, and
(b) an amino acid of formula AII 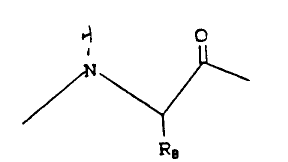
AA₃, which are each independently selected from the group consisting of
(a) a single bond, and
(b) an amino acid of formula AIII 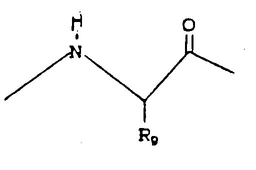
wherein R₈ and R₉ are each independently selected from the group consisting of
(a) hydrogen,
(b) substituted C₁₋₆ alkyl, wherein the substituent is selected from
   (1) hydrogen,
   (2) hydroxy,
   (3) halo,
   (4) -S-C₁₋₄ alkyl
   (5) -SH
   (6) C₁₋₆ alkylcarbonyl,
   (7) carboxy,
   (8) 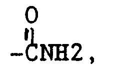
   (9) amino carbonyl amino,
   (10) C₁₋₄ alkylamino, wherein the alkyl moiety is substituted with hydrogen or hydroxy, and the amino is substituted with hydrogen or CBZ,
   (11) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl.

One class of this genus is the compounds wherein:
R₁ is
(a) substituted C₁₋₆ alkyl, wherein the substituent is selected from
   (1) hydrogen,
   (2) hydroxy,
   (3) chloro or fluoro, and
(b) aryl C₁₋₆ alkyl wherein the aryl group is selected from the group consisting of
   (1) phenyl,
   (2) naphthyl,
   (3) pyridyl,
   (4) furyl,
   (5) thienyl,
   (6) thiazolyl,
   (7) isothiazolyl,
   (8) benzofuryl,
   (9) benzothienyl,
   (10) indolyl,
   (11) isooxazolyl, and
   (12) oxazolyl,
and mono and di-substituted C₆₋₁₀aryl as defined above in items (1) to (12) wherein the substitutents are independently C₁₋₄alkyl, halo, and hydroxy;
R₂ is -C≡N;
AA₁ is independently selected from the group consisting of
(a) a single bond, and
(b) an amino acid of formula AI 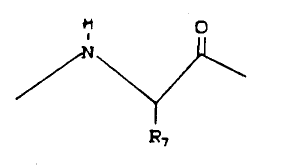
wherein R₇ is aryl C₁₋₆ alkyl
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl;
AA₂ is independently selected from the group consisting of
(a) a single bond, and
(b) an amino acid of formula AII 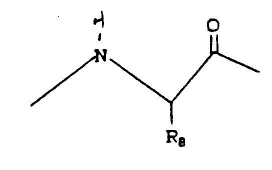
AA₃, which are each independently selected from the group consisting of
(a) a single bond, and
(b) an amino acid of formula AIII 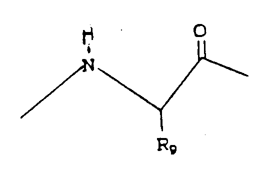
wherein R₈ and R₉ are each independently selected from the group consisting of
(a) hydrogen,
(b) C₁₋₆ alkyl, wherein the substituent is selected from
   (1) hydrogen,
   (2) hydroxy,
   (3) halo,
   (4) -S-C₁₋₄ alkyl
   (5) -SH
   (6) C₁₋₆ alkylcarbonyl,
   (7) carboxy,
   (8) 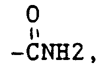
   (9) C₁₋₄ alkylamino, and C₁₋₄ alkyl amino wherein the alkyl moeity is substituted whith an hydroxy, and
   (10) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl, amino, C₁₋₆alkoxy, C₁₋₆alkylthio, and C₁₋₆alkylcarbonyl.

Within this class are the compounds wherein AA1, AA2 and AA3, are each independently selected from the group consisting of the L- and D- forms of the amino acids including glycine, alanine, valine, leucine, isoleucine, serine, threonine, aspartic acid, asparagine, glutamic acid, glutamine, lysine, hydroxy-lysine, histidine, arginine, phenylalanine, tyrosine, tryptophan, cysteine, methionine, ornithine, β-alanine, homoserine, homotyrosine, homophenylalanine and citrulline.

Alternatively, within this class are the subclass of compounds wherein
R₁ is C₁₋₃alkyl;
R₂ is -C≡N; and
R₈ and R₉ are each individually
(a) hydrogen,
(b) C₁₋₆alkyl,
(c) mercapto C₁₋₆alkyl,
(d) hydroxy C₁₋₆alkyl,
(e) carboxy C₁₋₆alkyl,
(g) aminocarbonyl C₁₋₆alkyl,
(h) mono - or di-C₁₋₆alkyl amino C₁₋₆alkyl,
(i) guanidino C₁₋₆alkyl,
(j) amino-C₁₋₆alkyl or N-substituted amino-C₁₋₆alkyl wherein the substituent is carbobenzoxy,
(k) carbamyl C₁₋₆alkyl, or
(l) aryl C₁₋₆alkyl, wherein the aryl group is selected from phenyl and indolyl, and the aryl group may be substituted with hydroxy, C₁₋₃ alkyl.

Within this sub-class are the compounds wherein:
R₁ is methyl;
R₂ is -C≡N;
R₈ is C₁₋₆alkyl; and
R₉ is
(a) hydrogen,
(b) C₁₋₆alkyl,
(d) benzyl,
(e) p-hydroxy-benzyl,
(f) N-carbobenzoxy-amino-(n-butyl),
(g) carbamylmethyl,
(h) carbamylethyl,
(i) indol-2-yl-methyl,
(j) substituted phenyl C₁₋₆alkyl, wherein the substituent is hydrogen, hydroxy, carboxy, or C₁₋₄alkyl,
(k) substituted indolyl C₁₋₆alkyl, wherein the substituent is hydrogen, hydroxy, carboxy, or C₁₋₄alkyl, or
(l) substituted imidazolyl C₁₋₆alkyl wherein the substituent is hydrogen, hydroxy, carboxy, or C₁₋₄alkyl.

Exemplifying the invention are the following compounds:
(a)N-(N-Acetyl-tyrosinyl-valinyl-alaninyl)-3-amino-3-cyanopropionic acid;
(b)N-(N-Acetyl-tyrosinyl-valinyl-ε-CBZ-lysinyl)-3-amino-3-cyanopropionic acid;
(c)N-(N-Acetyl-tyrosinyl-valinyl-lysinyl)-3-amino-3-cyanopropionic acid.

This invention also concerns to pharmaceutical composition and methods of treatment of interleukin-1 and interleukin-1β mediated or implicated disorders or diseases (as described above) in a patient (including man and/or mammalian animals raised in the dairy, meat, or fur industries or as pets) in need of such treatment comprising administration of interleukin-1β inhibitors of formula (I) as the active constituents.

Illustrative of these aspects, this invention concerns pharmaceutical compositions and methods of treatment of diseases selected from septic shock, allograft rejection, inflammatory bowel disease and rheumatoid arthritis in a patient in need of such treatment comprising:
administration of an interleukin-1β inhibitor of formula (I) as the active constituent.

Compounds of the instant invention are conveniently prepared using the procedures described generally below and more explicitly described in the Example section thereafter. 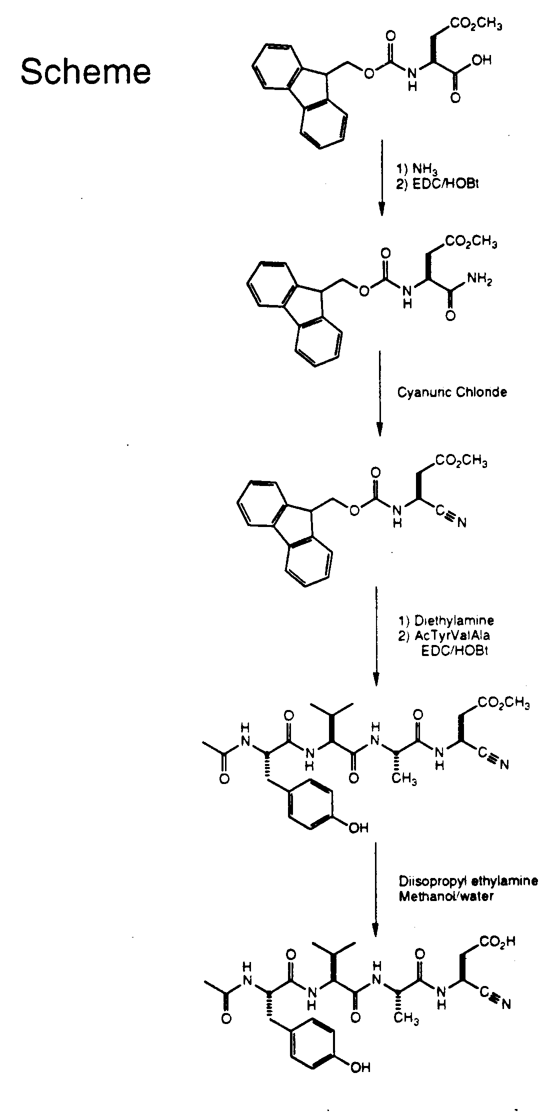
The reactions shown in the scheme proceed as follows. FMOC-Aspartic acid β-methyl ester is converted to its corresponding α-primary amide by treatment with ammonia followed by coupling with ethyl dimethylaminopropyl carbodiimide (EDC) in the presence of hydroxybenzotriazole (HOBt). The amide is then dehydrated by cyanuric chloride in dimethylformamide to give the nitrile. The FMOC protecting group is then removed with diethyl amine, and the resulting amine coupled to N-acetyltyrosinylvalinyl-alanine (AcTyrValAla) again using EDC and HOBt. Finally, the methyl ester is removed with diisopropylethyl amine in methanol water to afford the desired inhibitors.

The compounds of the instant invention of the formula (I), as represented in the Examples hereinunder shown to exhibit in vitro inhibitory activities with respect to interleukin-1β. In particular, these compounds have been shown to inhibit interleukin-1β converting enzyme from cleaving precusor interleukin-1β as to form active interleukin-1β at a Ki of less than 1 uM.

This invention also relates to a method of treatment for patients (including man and/or mammalian animals raised in the dairy, meat, or fur industries or as pets) suffering from disorders or diseases which can be attributed to IL-1/ICE as previously described, and more specifically, a method of treatment involving the administration of the IL-1/ICE inhibitors of formula (I) as the active constituents.

Accordingly, disease states in which the ICE inhibitors of Formula I may be useful as therapeutic agents include, but are not limited to, infectious diseases where active infection exists at any body site, such as meningitis and salpingitis; complications of infections including septic shock, disseminated intravascular coagulation, and/or adult respiratory distress syndrome; acute or chronic inflammation due to antigen, antibody, and/or complement deposition; inflammatory conditions including arthritis, cholangitis, colitis, encephalitis, endocarditis, glomerulonephritis, hepatitis, myocarditis, pancreatitis, pericarditis, reperfusion injury and vasculitis. Immune-based diseases which may be responsive to ICE inhibitors of Formula I include but are not limited to conditions involving T-cells and/or macrophages such as acute and delayed hypersensitivity, graft rejection, and graft-versus-host-disease; auto-immune diseases including Type I diabetes mellitus and multiple sclerosis. ICE inhibitors of Formula I may also be useful in the treatment of bone and cartilage resorption as well as diseases resulting in excessive deposition of extracellular matrix such as interstitial pulmonary fibrosis, cirrhosis, systemic sclerosis, and keloid formation. ICE inhibitors of Formula I may also be useful in treatment of certain tumors which produce IL 1 as an autocrine growth factor and in preventing the cachexia associated with certain tumors.

For the treatment the above mentioned diseases, the compounds of formula (I) may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intracisternal injection or infusion techniques. In addition to the treatment of warm-blooded animals such as mice, rats, horses, cattle, sheep, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Patents 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of formula (I) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compounds of Formula (I) are employed. (For purposes of this application, topical application shall include mouth washes and gargles.)

Dosage levels of the order of from about 0.05 mg to about 140 mg per kilogram of body weight per day are useful in the treatment of the above-indicated conditions (about 2.5 mg to about 7 gms. per patient per day). For example, inflammation may be effectively treated by the administration of from about 0.01 to 50 mg of the compound per kilogram of body weight per day (about 0.5 mg to about 3.5 gms per patient per day).

The amount of active ingredient that may be combined with the carrier materials to produce a single dosage form will vary depending upon the host treated and the particular mode of administration. For example, a formulation intended for the oral administration of humans may contain from 0.5 mg to 5 gm of active agent compounded with an appropriate and convenient amount of carrier material which may vary from about 5 to about 95 percent of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The following Examples are intended to illustrate the preparation of compounds of Formula I, and as such are not intended to limit the invention as set forth in the claims appended, thereto.

### EXAMPLE 1

### N-(N-Acetyl-tyrosinyl-valinyl-alaninyl)-3-amino-3-cyanopropionic acid.

### STEP A

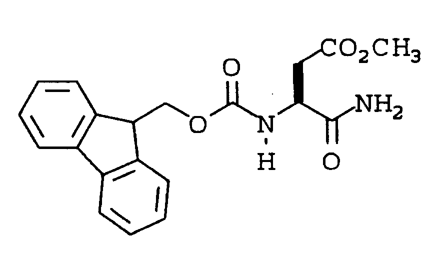

### N-Fluorenylmethyloxycarbonyl-aspartic acid β-methyl ester a-amide.

N-Fluorenylmethyloxycarbonyl-aspartic acid β-methyl ester (4.66 mmol) was disolved in ether and precipitated with ammonia gas. The solid was collected and washed several times with ether then dried in-vacuo. This product was disolved in 10 mL each of tetrahydrofuran and dimethylformamide, and hydroxybenzotriazole (HOBt, 944 mg, 6.99 mmol) was added. The solution was cooled to 0°C and ethyl dimethylaminopropyl carbodiimide (EDC, 982 mg, 5.12 mmol) was added. After 3 h at ambient temperature, the mixture was diluted with ethyl acetate and washed three times with 2 N hydrochloric acid and three times with saturated sodium bicarbonate. The organics were then dried over sodium sulfate, filtered, and concentrated to give a colorless solid. The product was crystallized from hot ethyl acetate to afford 1.044 g of the title product as a colorless, crystalline solid: ¹H NMR (400 MHz, CD₃OD) δ 7.79 (d, 2H, J = 7.47 Hz, Ar-H), 7.65 (d, 2H, J = 7.47 Hz, Ar-H), 7.38 (t, 2H, J = 7.38 Hz, Ar-H), 7.30 (t, 2H, J = 7.43 Hz, Ar-H), 4.51 (dd, 1H, J = 5.44, 7.89 Hz, CHN), 4.39 (m, 2H, CH₂O), 4.22 (t, 1H, J = 6.96 Hz, CHCH₂O), 3.66 (s, 3H, CH₃), 2.84 (dd, 1H, J = 5.67, 16.46 Hz, CHHCO₂), 2.68 (dd, 1H, J = 8.21, 16.46 Hz, CHHCO₂).

### STEP B

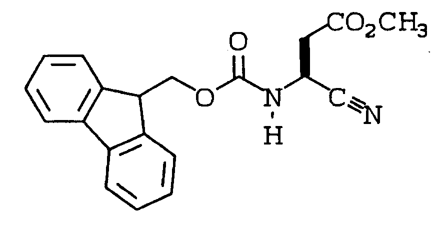

### N-Fluorenylmethyloxycarbonyl-3-amino-3-cyanopropionic acid methyl ester.

To a solution of N-Fluorenylmethyloxycarbonyl-aspartic acid β-methyl ester α-amide (1.044 g, 2.83 mmol) in 20 mL of dimethylformamide, which had been allowed to stand for at least three days in the presence of 3Å and 13X molecular sieves, was added cyanuric chloride (784 mg, 4.25 mmol). After 15 min, the mixture was diluted with ethyl acetate and washed three times with 2 N hydrochloric acid and three times with saturated sodium bicarbonate. The organics were dried over sodium sulfate, filtered, and concentrated to give a colorless solid. The product was purified by MPLC on silica-gel (22x300 mm column, eluting with 30% ethyl acetate in hexane) to afford 920.2 mg of the title compound as a colorless solid: ¹H NMR (400 MHz, CD₃OD) δ 7.79 (d, 2H, J = 7.51 Hz, Ar-H), 7.63 (d, 2H, J = 7.37 Hz, Ar-H), 7.38 (t, 2H, J = 7.43 Hz, Ar-H), 7.30 (t, 2H, J = 7.47 Hz, Ar-H), 4.88 (m, 1H, CHN (Partially obscured by CD₃OH)), 4.42 (d, 2H, J = 6.54 Hz, CH₂O), 4.22 (t, 1H, J = 6.31 Hz, CHCH₂O), 3.71 (s, 3H, CH₃), 2.92 (ABX, 2H, CH₂CO₂).

### STEP C

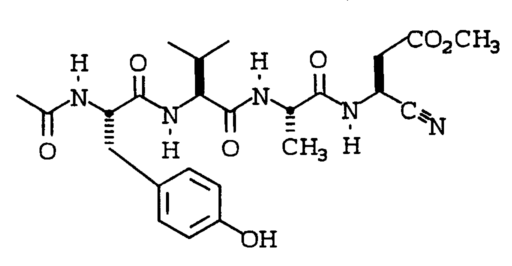

### N-(N-Acetyltyrosinyl-valinyl-alaninyl)-3-amino-3-cyanopropionic acid methyl ester.

N-Fluorenylmethyloxycarbonyl-3-amino-3-cyanopropionic acid methyl ester (900 mg) was disolved in 20 mL of diethylamine, stirred for 1 h, and concentrated. The residue was purified by MPLC on silica-gel (22x300 mm column, eluting with a gradient of dichloromethane to 0.25% ammonia and 2.5% methanol in dichloromethane) to give a pale yellow oil. To a solution of 101.5 mg (0.792 mmol) of this oil in 5 mL of dimethylformamide at 0 °C, was added N-acetyltyrosinyl-valinyl-alanine (383 mg, 0.792 mmol) followed by hydroxybenzotriazole (214 mg, 1.58 mmol) and finally ethyl dimethylaminopropyl carbodiimide (167 mg, 0.871 mmol). After 16 h at ambient temperature, the mixture was diluted with ethyl acetate and washed three times with 2 N hydrochloric acid, three times with dilute sodium bicarbonate, and twice with water. The organics were dried over sodium sulfate, filtered, and concentrated. The product was purified by MPLC on silica-gel (22x300 mm column, eluting with a gradient of dichloromethane to 20% methanol in dichloromethane) to afford the title compound as a colorless solid: ¹H NMR (400 MHz, CD₃OD) δ 7.03 (d, 2H, J = 8.62 Hz, Ar-H), 6.67 (d, 2H, J = 8.53 Hz, Ar-H), 5.08 (t, 1H, J = 6.73 Hz, CHCN), 4.56 (dd, 2H, J = 5.77, 8.95 Hz, CHNAc), 4.26 (q, 1H, J = 7.19 Hz, CHCH₃), 4.13 (d, 1H, J = 7.24 Hz, CHCH(CH₃)₂), 3.72 (s, 3H, CH₃O), 3.1-2.7 (m, 4H, CH₂CO₂, CH₂Ar), 2.03 (m, 1H, CH(CH₃)₂), 1.93 (s, 3H, CH₃CON), 1.35 (d, 3H, J = 7.15 Hz, CHCH₃), 0.93 (t, 6H, J = 6.96 Hz, CH(CH₃)₂).

### STEP D

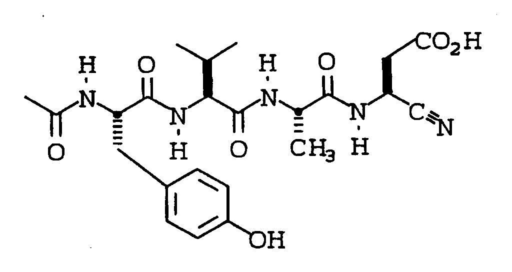

### N-(N-Acetyltyrosinyl-valinyl-alaninyl)-3-amino-3-cyanopropionic acid.

To a solution of N-(N-Acetyltyrosinyl-valinyl-alaninyl)-3-amino-3-cyanopropionic acid methyl ester (26.6 mg) in three mL each of methanol and water was added 600 µL of freshly distilled diisopropyl ethylamine. After three hours at ambient temperature, the mixture was concentrated. The residue was purified by MPLC on silica-gel (7x250 mm column, eluting with a gradient of dichloromethane to 8% formic acid and 32% methanol in dichloromethane) to afford 20 mg of the title compound as a colorless solid: ¹H NMR (400 MHz, CD₃OD) δ 7.04 (d, 2H, J = 8.53 Hz, Ar-H), 6.67 (d, 2H, J = 8.58 Hz, Ar-H), 5.03 (t, 1H, J = 6.77 Hz, CHCN), 4.57 (dd, 2H, J = 5.77, 8.99 Hz, CHNAc), 4.28 (q, 1H, J = 7.20 Hz, CHCH₃), 4.14 (d, 1H, J = 7.29 Hz, CHCH(CH₃)₂), 2.99 (dd, 1H, J = 5.80, 15.56 Hz, CHHCO₂), 2.9-2.7 (m, 3H, CHHCO₂, CH₂Ar), 2.04 (m, 1H, CH(CH₃)₂), 1.90 (s, 3H, CH₃CON), 1.36 (d, 3H, J = 6.60 Hz, CHCH₃), 0.93 (t, 6H, J = 7.60 Hz, CH(CH₃)₂).
The following additional compounds are made in an anologous manner:
N-(N-Acetyl-phenylalaninyl-valinyl-alaninyl)-3- amino-3-cyanopropionic acid;
N-(3-phenylpropionyl-valinyl-alaninyl)-3-amino-3-cyanopropionic acid; and
N-(3-(4-hydroxyphenyl)-valinyl-alaninyl)-3-amino-3-cyanopropionic acid.
N-(N-Acetyl-phenylalaninyl-valinyl-ε-CBZ-lysinyl)-3-amino-3-cyanopropionic acid;
N-(3-phenylpropionyl-valinyl-ε-CBZ-lysinyl)-3-amino-3-cyanopropionic acid; and
N-(3-(4-hydroxyphenyl)-propionyl-valinyl-ε-CBZ-lysinyl)-3-amino-3-cyanopropionic acid.
N-(N-Acetyl-phenylalaninyl-valinyl-lysinyl)-3- amino-3-cyanprpopionic acid;
N-(3-phenylpropionyl-valinyl-lysinyl)-3-amino-3-cyanopropionic acid; and
N-(3-(4-hydroxyphenyl)-propionyl-valinyl-lysinyl)-3-amino-3-cyanopropionic acid.

## Claims

1. A compound of formula I 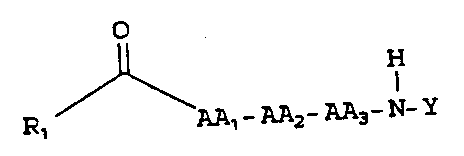 or a pharmaceutically acceptable salt thereof thereof: wherein Y is: 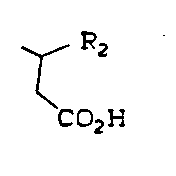 R₁ is
(a) substituted C₁₋₁₂ alkyl, wherein the substituent is selected from
(1) hydrogen,
(2) hydroxy,
(3) halo, and
(4) C₁₋₆ alkylcarbonyl;
(b) aryl C₁₋₆ alkyl wherein the aryl group is selected from the group consisting of:
(1) phenyl,
(2) naphthyl,
(3) pyridyl,
(4) furyl, (5) thienyl,
(6) thiazolyl,
(7) isothiazolyl,
(8) imidazolyl,
(9) benzimidazolyl,
(10) pyrazinyl,
(11) pyrimidyl,
(12) quinolyl,
(13) isoquinolyl,
(14) benzofuryl,
(15) benzothienyl,
(16) pyrazolyl,
(17) indolyl,
(18) purinyl,
(19) isoxazolyl, and
(20) oxazolyl,
and mono and di-substituted aryl as defined above in items (1) to (20) wherein the substitutents are independently C₁₋₆alkyl, halo, hydroxy, C₁₋₆alkyl amino, C₁₋₆alkoxy, C₁₋₆alkylthio, and C₁₋₆alkylcarbonyl;
R₂ is -C≡N;
AA₁ is selected from the group consisting of
(a) a single bond, and
(b) an amino acid of formula AI 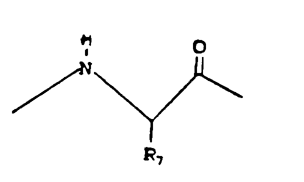
wherein R₇ selected from the group consisting of
(a) hydrogen,
(b) substituted C₁₋₆ alkyl, wherein the substituent is selected from
(1) hydrogen,
(2) hydroxy,
(3) halo,
(4) -S-C₁₋₄ alkyl,
(5) -SH
(6) C₁₋₆ alkylcarbonyl,
(7) carboxy,
(8) 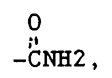
(9) amino carbonyl amino,
(10) C₁₋₄ alkylamino, wherein the alkyl moeity is substituted with hydrogen or hydroxy, and the amino is substituted with hydrogen or CBZ,
(11) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl,
wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl;
AA₂ is selected from the group consisting of
(a) a single bond, and
(b) an amino acid of formula AII 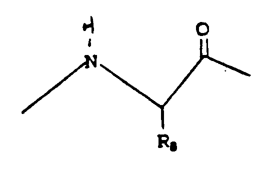
AA₃ is selected from the group consisting of
(a) a single bond, and
(b) an amino acid of formula AIII 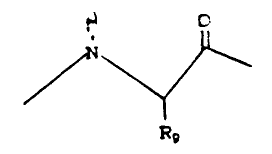
wherein R₈ and R₉ are each independently selected from the group consisting of
(a) hydrogen,
(b) substituted C₁₋₆ alkyl, wherein the substituent is selected from
(1) hydrogen,
(2) hydroxy,
(3) halo,
(4) -S-C₁₋₄ alkyl,
(5) -SH
(6) C₁₋₆ alkylcarbonyl,
(7) carboxy,
(8) 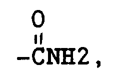
(9) amino carbonyl amino,
(10) C₁₋₄ alkylamino, wherein the alkyl moeity is substituted with hydrogen or hydroxy, and the amino is substituted with hydrogen or CBZ,
(11) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl.

2. A compound of Claim 1 wherein:
R₁ is
(a) substituted C₁₋₆ alkyl, wherein the substituent is selected from
(1) hydrogen,
(2) hydroxy, and
(3) chloro or fluoro,
(b) aryl C₁₋₆ alkyl wherein the aryl group is selected from the group consisting of
(1) phenyl,
(2) naphthyl,
(3) pyridyl,
(4) furyl,
(5) thienyl,
(6) thiazolyl,
(7) isothiazolyl,
(8) benzofuryl,
(9) benzothienyl,
(10) indolyl,
(11) isooxazolyl, and
(12) oxazolyl,
and mono and di-substituted C₆₋₁₀aryl as defined above in items (1) to (12) wherein the substitutents are independently C₁₋₄alkyl, halo, and hydroxy;
R₂ is -C≡N;
AA₁ is selected from the group consisting of
(a) a single bond, and
(b) an amino acid of formula AI 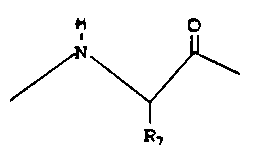
wherein R₇ is selected from the group consisting of
(a) hydrogen,
(b) substituted C₁₋₆ alkyl, wherein the substituent is selected from
(1) hydrogen,
(2) hydroxy,
(3) halo,
(4) -S-C₁₋₄ alkyl
(5) -SH
(6) C₁₋₆ alkylcarbonyl,
(7) carboxy,
(8) 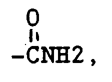
(9) C₁₋₄ alkylamino, and C₁₋₄ alkylamino wherein the alkyl moeity is substituted whith an hydroxy, and
(10) guanidino, and
(c) aryl C₁₋₆ alkyl, wherein the aryl group is elected from the group consisting of
(1) phenyl,
(2) naphthyl,
(3) pyridyl,
(4) furyl,
(5) thienyl,
(6) thiazolyl,
(7) isothiazolyl,
(8) benzofuryl,
(9) benzothienyl,
(10) indolyl,
(11) isooxazolyl, and
(12) oxazolyl,
and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl;
AA₂ is selected from the group consisting of
(a) a single bond, and
(b) an amino acid of formula AII 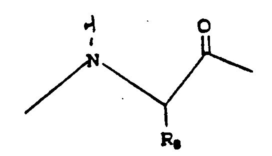
wherein R₈ is selected from the group consisting of
(a) hydrogen,
(b) substituted C₁₋₆ alkyl, wherein the substituent is selected from
(1) hydrogen,
(2) hydroxy,
(3) halo,
(4) -S-C₁₋₄ alkyl
(5) -SH
(6) C₁₋₆ alkylcarbonyl,
(7) carboxy,
(8) 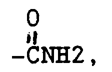
(9) C₁₋₄ alkylamino, and C₁₋₄ alkylamino wherein the alkyl moeity is substituted whith an hydroxy, and
(10) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl; and
AA₃ is selected from the group consisting of
(a) a single bond, and
(b) an amino acid of formula AIII 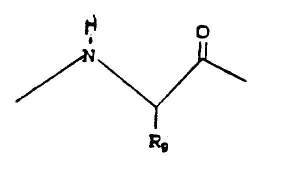
R₉ is selected from the group consisting of
(a) hydrogen,
(b) substituted C₁₋₆ alkyl, wherein the substituent is selected from
(1) hydrogen,
(2) hydroxy,
(3) halo,
(4) -S-C₁₋₄ alkyl
(5) -SH
(6) C₁₋₆ alkylcarbonyl,
(7) carboxy,
(8) 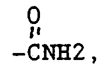
(9) C₁₋₄ alkylamino, and C₁₋₄ alkylamino wherein the alkyl moeity is substituted whith an hydroxy, and
(10) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl.

3. A compound according to Claim 2 wherein
AA₁ is a single bond or an amino acid of formula AI 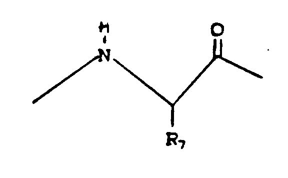 wherein R₇ is aryl C₁₋₆ alkyl
wherein aryl is defined as
(1) phenyl,
(2) naphthyl,
(3) pyridyl,
(4) furyl,
(5) thienyl,
(6) thiazolyl,
(7) isothiazolyl,
(8) benzofuryl,
(9) benzothienyl,
(10) indolyl,
(11) isooxazolyl, and
(12) oxazolyl,
and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl;
AA₂ is an amino acid of the fromula AII 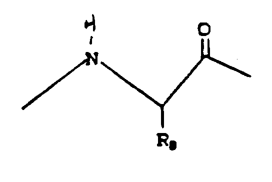 wherein R₈ is selected from the group consisting of
(a) hydrogen,
(b) substituted C₁₋₆ alkyl, wherein the substituent is selected from
(1) hydrogen,
(2) hydroxy,
(3) halo,
(4) -S-C₁₋₄ alkyl
(5) -SH
(6) C₁₋₆ alkylcarbonyl,
(7) carboxy,
(8) 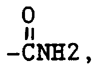
(9) C₁₋₄ alkylamino, and C₁₋₄ alkylamino wherein the alkyl moeity is substituted whith an hydroxy, and
(10) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁₋₆alkylthio, and C₁₋₆alkylcarbonyl; and
AA₃ is an amino acid of formula AIII 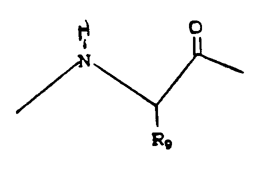 wherein R₉ is selected from the group consisting of
(a) hydrogen,
(b) substituted C₁₋₆ alkyl, wherein the substituent is selected from
(1) hydrogen,
(2) hydroxy,
(3) halo,
(4) -S-C₁₋₄ alkyl
(5) -SH
(6) C₁₋₆ alkylcarbonyl,
(7) carboxy,
(8) 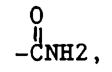
(9) C₁₋₄ alkylamino, and C₁₋₄ alkylamino wherein the alkyl moeity is substituted whith an hydroxy, and
(10) guanidino, and
(c) aryl C₁₋₆ alkyl,
wherein aryl is defined as immediately above, and wherein the aryl may be mono and di-substituted, the substituents being each independently C₁₋₆alkyl, halo, hydroxy, C₁₋₆alkyl amino, C₁₋₆alkoxy, C₁-₆alkylthio, and C₁₋₆alkylcarbonyl.

4. A compound according to Claim 3 wherein
R₁ is C₁₋₃alkyl or aryl C₁₋₆ alkyl wherein aryl is phenyl, naphthyl, thienyl, or benzothienyl; R₈ and R₉ are each individually
(a) hydrogen,
(b) C₁₋₆alkyl,
(c) mercapto C₁₋₆alkyl,
(d) hydroxy C₁₋₆alkyl,
(e) carboxy C₁₋₆alkyl,
(g) aminocarbonyl C₁₋₆alkyl,
(h) mono - or di-C₁₋₆alkyl amino C₁₋₆alkyl,
(i) guanidino C₁₋₆alkyl,
(j) amino-C₁₋₆alkyl or N-substituted amino-C₁₋₆alkyl wherein the substituent is carbobenzoxy, or
(k) aryl C₁₋₆alkyl, wherein the aryl group is selected from phenyl and indolyl, and the aryl group is substituted with hydrogen, hydroxy, C₁₋₃ alkyl.

5. A compound According to Claim 4 wherein:
R₁ is methyl or phenyl C₁₋₆ alkyl or hydroxy-phenyl C₁₋₆ alkyl;
AA1 is a single bond or an amino acid of formula AI 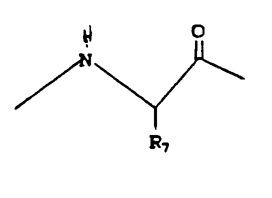 wherein R₇ is
(a) C₁₋₆alkyl;
(b) substituted phenyl C₁₋₃alkyl, wherein the substituent is hydrogen, hydroxy, carboxy, or C₁₋₄alkyl; or
(c) indolyl methyl;
R₈ is C₁₋₆alkyl; and
R₉ is
(a) hydrogen,
(b) C₁₋₆alkyl,
(c) amino C₁₋₄alkyl,
(d) N-carbobenzoxy-amino-(n-butyl),
(e) carbamylmethyl,
(f) indol-2-yl-methyl, or
(g) substituted phenyl C₁₋₃alkyl, wherein the substituent is hydrogen or hydroxy.

6. A compound according to Claim 5 wherein R₇ is
(a) C₁₋₆alkyl;
(b) substituted phenyl C₁₋₃alkyl, wherein the substituent is hydrogen or hydroxy; or
(c) indolyl methyl.

7. A compound according to Claim 6 wherein
R₁ is methyl or phenyl C₁₋₆ alkyl or hydroxy-phenyl C₁₋₆ alkyl;
AA₁ is a single bond or tyrosinyl, homotyrosinyl, phenylalaninyl, homophenylalaninyl or tryptophanyl;
AA₂ is 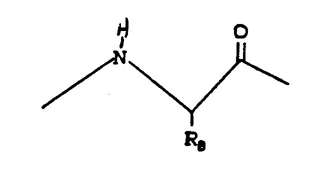 wherein R8 is C₁₋₄ alkyl; and
AA₃ is alaninyl, lysinyl or ε-CBZ-lysinyl.

8. A compound according to Claim 7 wherein
R₁ is phenyl C₁₋₆ alkyl or hydroxy-phenyl C₁₋₆ alkyl;
AA1 is a single bond;
AA2 is 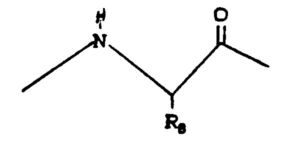 wherein R₈ is C₁₋₄ alkyl; and
AA3 is alaninyl, lysinyl or ε-CBZ-lysinyl.

9. A compound according to Claim 8 wherein
R₁ is phenyl ethyl or hydroxy-phenyl ethyl.

10. A compound according to Claim 9 wherein
R₁ is methyl;
AA₁ is tyrosinyl, homotyrosinyl, phenylalaninyl, homophenylalaninyl or tryptophanyl;
AA2 is 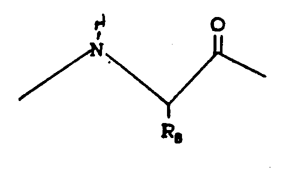 wherein R₈ is C₁₋₄ alkyl; and
AA₃ is alaninyl, lysinyl or ε-CBZ-lysinyl.

11. A compound according to Claim 10 wherein
R₁ is methyl;
AA₁ is tyrosinyl;
AA₂ is leucinyl; and
AA₃ is alaninyl, lysinyl or ε-CBZ-lysinyl; or
R₁ is methyl;
AA₁ is tyrosinyl;
AA₂ is valinyl; and
AA₃ is alaninyl, lysinyl or ε-CBZ-lysinyl; or
R₁ is methyl;
A₁ is tyrosinyl;
AA₂ is isoleucinyl; and
AA₃ is alaninyl, lysinyl or ε-CBZ-lysinyl.

12. A compound selected from the group consisting of:
(a)N-(N-Acetyl-tyrosinyl-valinyl-lysinyl)-3-amino-3-cyanopropionic acid;
(b)N-(N-Acetyl-tyrosinyl-valinyl-ε-CBZ-lysinyl)-3-amino-3-cyanopropionic acid;
(c)N-(N-Acetyl-tyrosinyl-valinyl-alaninyl)-3-amino-3-cyanopropionic acid;
(d)N-(N-Acetyl-phenylalaninyl-valinyl-lysinyl)-3- amino-3-cyanopropionic acid;
(e)N-(N-Acetyl-phenylalaninyl-valinyl-ε-CBZ-lysinyl)-3-amino-3-cyanopropionic acid;
(f)N-(N-Acetyl-phenylalaninyl-valinylalaninyl)-3- amino-3-cyanopropionic acid;
(g)N-(3-phenylpropionyl-valinyl-lysinyl)-3-amino-3-cyanopropionic acid;
(h)N-(3-phenylpropionyl-valinyl-ε-CBZ-lysinyl)-3-amino-3-cyanopropionic acid;
(i)N-(3-phenylpropionyl-valinyl-alaninyl)-3-amino-3-cyanopropionic acid;
(j)N-(3-(4-hydroxyphenyl)-propionyl-valinyl-lysinyl)-3-amino-3-cyanopropionic acid;
(k)N-(3-(4-hydroxyphenyl)-propionyl-valinyl-ε-CBZ-lysinyl)-3-amino-3-cyanopropionic acid; and
(1)N-(3-(4-hydroxyphenyl)-valinyl-alaninyl)-3-amino-3-cyanopropionic acid.

13. A compound according to Claim 6 wherein
R₁ is phenylmethyl, phenylethyl, phenylpropyl, phenylbutyl, phenylpentyl, or phenylhexyl wherein the methyl, ethyl, propyl, butyl, pentyl, or hexyl, wherein the alkyl portion subsitiuted and wherein the substitutent is hydrogen, C₁₋₃ alkyl, C₁₋₃ alkoxy, C₁₋₃ alkylcarbonyl, C₁₋₃ alkylthio, C₁₋₃ alkylamino, halo or hydroxy;
AA₁ is a single bond;
AA₂ is a single bond; and
AA₃ is alaninyl, lysinyl or ε-CBZ-lysinyl.

14. A pharmaceutical composition for treatment interleukin-1 mediated disorders or diseases in a patient in need of such treatment comprising administration of an interluekin-1β inhibitor according to Claim 1 as the active constituent.
